# EUROPÄISCHE PATENTSCHRIFT

(11) **EP 4 117 746 B1**
(45) Veröffentlichungstag und Bekanntmachung des Hinweises auf die Patenterteilung: **20.08.2025**
(21) Anmeldenummer: 21712091.4
(22) Anmeldetag: 11.03.2021
(51) Int. Cl.: A61M 1/14, G16H 20/40, G16H 40/63

(54) **MEDIZINISCHES GERÄT SOWIE VERFAHREN ZUR KOMMUNIKATION FÜR EIN MEDIZINISCHES GERÄT**
MEDICAL APPARATUS AND METHOD OF COMMUNICATION FOR A MEDICAL APPARATUS
APPAREIL MÉDICAL ET PROCÉDÉ DE COMMUNICATION POUR UN APPAREIL MÉDICAL

(30) Priorität: 11.03.2020 DE 102020106643
(43) Veröffentlichungstag der Anmeldung: 18.01.2023
(73) Patentinhaber: Fresenius Medical Care Deutschland GmbH, 61352 Bad Homburg (DE)
(72) Erfinder: SCHRÖRS, Alexander, 60389 Frankfurt a. M. (DE); SCHRÖDER, Robert, 64291 Darmstadt (DE); KEUNE, Lucas, 52072 Aachen (DE); SCHREIBER, Lena, 61352 Bad Homburg (DE)
(74) Vertreter: Herrmann, Uwe
(86) Internationale Anmeldenummer: PCT/EP2021/056127
(87) Internationale Veröffentlichungsnummer: WO 2021/180827

(56) Entgegenhaltungen:
- EP-A1- 2 740 413
- US-A1- 2011 301 439
- US-A1- 2016 284 198

## Beschreibung

Die vorliegende Erfindung betrifft ein medizinisches Gerät sowie ein Verfahren zur Kommunikation für ein medizinisches Gerät und vorzugsweise mindestens eine weitere andere Vorrichtung.

Herkömmliche medizinische Geräte, wie beispielsweise extrakorporale Blutbehandlungsgeräte, insbesondere Dialysegeräte, zeichnen sich durch eine nur begrenzte Kommunikationsfähigkeit aus.

Die US2016/284198A1 offenbart ein Verfahren nur Einstellung eines Tons bei einem Medizingerät. Aus der EP2740413A1 ist ein Kopplungsverfahren für eine Steuereinheit und ein Medizingerät bekannt. Die US2011/301439A1 offenbart ein System zur persönlichen Gesundheitsüberwachung.

Insbesondere in Situationen, in welchen mehrere derartige medizinische Geräte koordiniert betrieben werden müssen, führt dies zu Herausforderungen.

So wird beispielsweise in einer Dialysestation üblicherweise eine Vielzahl von Dialysegeräten zeitlich getaktet betrieben. Da alle Geräte die gleichen Vor-/Nachbereitungs- und Betriebsprozesse möglichst zeitgleich durchführen sollen, ist eine Synchronisation der Geräte erforderlich.

Ohne eine Kommunikationsmöglichkeit zwischen den Geräten wird der Betrieb einer Gruppe von Geräten jedoch herkömmlicherweise als Sequenzierung von manuellen Prozesswiederholungen realisiert. Dafür muss das Personal von Gerät zu Gerät laufen und an jedem einzeln durch eine manuelle Eingabe den gleichen Prozess initiieren. Das führt nicht nur zu einer physischen (Laufwege) und psychischen (Prozesswiederholung) Belastung des Personals, sondern auch zu ungewünschten Verzögerungen im Betriebsablauf.

Typischerweise initiiert das Personal vor dem Beginn des Behandlungsbetriebs alle Maschinen einzeln nacheinander und leitet dann, nach der Beendigung des einzelnen Vorbereitungsschritts durch das jeweilige Gerät, bei jedem Gerät den nächsten Prozess ein. Da die einzelnen Prozesse vom Gerät abhängig unterschiedlich lange dauern können, ist der resultierende Ablauf oft unkoordiniert und ineffizient.

Um diese Probleme aufgrund der mangelnden Kommunikationsfähigkeit der medizinischen Geräte zu beheben, sind aus dem Stand der Technik verschiedene Lösungsansätze bekannt.

Beispielsweise soll die Kommunikationsfähigkeit erhöht werden, indem medizinische Gräte, z.B. Dialysegeräte, mit einer Netzwerkschnittstelle ausgerüstet bzw. nachgerüstet werden, über die Daten z. B. per Intranet vom/zum Gerät übertragen werden.

Bei älteren Geräten, die den Großteil aller genutzten Dialysegeräte ausmachen, muss diese Netzwerkschnittstelle nachgerüstet werden, was technisch, regulatorisch und finanziell einen beträchtlichen Aufwand darstellt und nicht immer möglich ist.

Der vorliegenden Erfindung liegt somit die Aufgabe zugrunde, die aus dem Stand der Technik bekannten Probleme abzumildern oder gar ganz zu beseitigen. Konkret sollen ein medizinisches Gerät mit einer verbesserten Kommunikationsfähigkeit sowie ein Verfahren zur Kommunikation eines medizinischen Geräts mit anderen medizinischen Geräten oder auch nicht-medizinischen Vorrichtungen bereitgestellt werden.

Diese Aufgabe wird durch ein medizinisches Gerät gemäß Anspruch 1 sowie durch ein Verfahren gemäß Anspruch 9 gelöst.

Ein erfindungsgemäßes medizinisches Gerät, insbesondere ein Gerät zur extrakorporalen Blutbehandlung, weist eine Ausgabeeinheit zur Entsendung von Systemtönen zur Kommunikation mit einem Benutzer auf, wobei die Ausgabeeinheit zusätzlich dazu ausgelegt ist, Mitteilungstöne zur Kommunikation mit mindestens einer anderen Vorrichtung auszusenden.

In anderen Worten wird die bestehende Hardware, also die Ausgabeeinheit, genutzt, um neben der Kommunikation mit einem Benutzer auch eine Kommunikation mit anderen medizinischen Geräten oder auch nicht-medizinischen Vorrichtungen auszuführen. Durch diese doppelte Nutzung der bestehenden Ausgabeeinheit entfällt die Notwendigkeit einer aufwändigen Nachrüstung.

Hierbei wird sich zunutze gemacht, dass viele gängige Dialysegeräte mit einer Ausgabeeinheit, beispielsweise in Form von einem Lautsprecher oder Lautsprechern, ausgestattet sind. Dies ist regulatorisch oft vorgeschrieben, damit das Personal per Ton alarmiert werden kann.

Damit die Funktion des Lautsprechers sichergestellt werden kann, sind die Geräte auch mit einer Empfangseinheit, insbesondere mit einem Mikrofon, ausgestattet, das u.a. auf alle Systemtöne sensitiv ist.

In anderen Worten sollen somit erfindungsgemäß diese bereits bestehenden Einheiten (Mikrophon und Lautsprecher) und die bereits etablierten Systemtöne (z.B. Informationstöne, Meldungstöne, Alarmtöne, Test-Töne) genutzt werden, um eine zusätzliche Kommunikationsschnittstelle ohne eine Aufrüstung /Nachrüstung der Geräte zu realisieren.

Dafür werden erfindungsgemäß vorzugsweise Daten, die zwischen den Geräten kommuniziert werden sollen, verschlüsselt und als Tonsequenz (auch als Mitteilungston bezeichnet) codiert. Diese verschlüsselten Mitteilungen werden vorzugsweise zeitgleich zu den emittierten Systemtönen von derselben Ausgabeeinheit bzw. vom gleichen Lautsprecher ausgesendet und von der Empfangseinheit (z.B. Mikrophon) eines anderen Geräts empfangen.

Vorzugsweise wird die Frequenz des Mitteilungstons so in Beziehung zur Frequenz des Systemtons gesetzt, dass die zusätzliche Mitteilung / der Mitteilungston vom menschlichen Ohr nicht wahrgenommen werden kann. Dieses Verfahren dient der Maskierung des Mitteilungstons.

Eine andere Art der Maskierung, die nicht Teil der beanspruchten Erfindung ist, ist die zeitliche Maskierung, bei der am Ende des Systemtons ein zusätzlicher, vorzugsweise relativ zu dem systemton kurzer und leiser Mitteilungston, der die zusätzliche Datenmitteilung enthält, angehängt wird.

Es hat sich in der Praxis als vorteilhaft erwiesen, wenn hierfür bei einem erfindungsgemäßen medizinischen Gerät die auf dem Gerät gespeicherte Datenbank der Systemtöne um die Mitteilungstöne, sowie deren Anpassung zur Maskierung innerhalb eines beliebigen Systemtons, erweitert ist. In anderen Worten enthält die Datenbank eines erfindungsgemäßen medizinischen Geräts somit nicht nur die herkömmlichen Systemtöne, sondern auch eine Anzahl an Mitteilungstönen. Zudem verfügt das medizinische Gerät über eine entsprechende Software, die es dem Gerät ermöglicht, die Mitteilungstöne entsprechend zu verschlüsseln bzw. empfangene Mitteilungstöne zu entschlüsseln und / oder zu maskieren bzw. zu demaskieren.

Vorzugsweise sind die von einem erfindungsgemäßen medizinischen Gerät ausgegebenen Systemtöne für das menschliche Gehör wahrnehmbar und die Mitteilungstöne, allein oder in Kombination mit den Systemtönen, sind für das menschliche Gehör nicht wahrnehmbar, sodass Benutzer durch die Kommunikation zwischen den Geräten und Vorrichtungen nicht gestört werden bzw. diese gar nicht wahrnehmen.

Vorzugsweise ist die Empfangseinheit eines erfindungsgemäßen medizinischen Geräts dazu ausgelegt, die Mitteilungstöne von mindestens einer anderen Vorrichtung, welche ein medizinisches oder auch ein nicht-medizinisches Gerät (z.B. ein Mobiltelefon oder Tablet) ist, zu empfangen und vorzugsweise zu entschlüsseln bzw. zu demaskieren.

Daten, die in einer solchen maskierten Mitteilung bzw. einem Mitteilungston einzeln oder in Kombination enthalten sein können, sind u.a.:
eine Geräte-ID, eine Standort-ID, eine ID einer Fehlermeldung, eine ID eines laufenden und / oder zuletzt abgeschlossenen und / oder als nächstes anstehenden Prozesses, eine Mitteilung bezüglich des Status des Prozesses, z. B. Daten bezüglich des zeitlichen Fortschritts der Reihenfolge einer Warteschlange mehrerer Geräte oder ein Auslöser / Trigger für Handlungsanweisungen für andere Geräte. Diese Aufzählung ist jedoch nur beispielhaft und die Art der übermittelten Daten ist völlig beliebig.

All diese Daten bzw. Informationen können erfindungsgemäß inklusive Redundanzen innerhalb eines Mitteilungstons codiert werden, der gängige Systemtöne zeitlich nicht überdauert.

Es können auch zusätzlich Tonsequenzen inkludiert werden, die im Rahmen natürlich generierter Töne nicht vorkommen, um eine Verwechslung des Mitteilungstons mit Umgebungsgeräuschen zu vermeiden.

Die Empfangseinheit bzw. das Mikrofon eines Geräts nimmt beide Anteile, den Systemton und den vorzugsweise maskierten Mitteilungston, des emittierten Tons auf, und die beiden Anteil werden vorzugsweise innerhalb eines Gerätecomputers (welcher vorzugsweise eine Auswerteeinheit und einer Steuerungseinheit aufweist) des empfangenden Geräts getrennt verarbeitet: Der Systemton wird wie herkömmlicherweise üblich verarbeitet, z.B. als Selbsttest, und der maskierte Meldungston wird decodiert und dann je nach Inhalt prozessiert.

Gemäß einer vorteilhaften Ausführungsform ist eine Steuerungseinheit eines erfindungsgemäßen medizinischen Geräts dazu ausgelegt, das medizinische Gerät in Übereinstimmung mit einem von der Empfangseinheit empfangenen Mitteilungston anzusteuern.

Vorzugsweise steuert die Steuerungseinheit das medizinische Gerät dazu an, den empfangenen Mitteilungston zumindest teilweise auszugeben, einen vorbestimmten und vorzugsweise in einer Datenbank abgelegten Prozess zu initiieren oder zu beenden, Daten, insbesondere Daten bezüglich des Gerätezustands, als vorzugsweise maskierten Mitteilungston auszugeben, die Empfangseinheit auszuschalten oder Mitteilungstöne zu ignorieren oder einen für das menschliche Gehör wahrnehmbaren Ton zum Zwecke der Alarmierung auszugeben. Diese Aufzählung ist jedoch nur beispielhaft und andere Handlungen sind denkbar.

Zur Ausführung derartiger Handlungen wird die Software des gerätesteuernden Computers bzw. der Steuerungseinheit eines erfindungsgemäßen medizinischen Geräts erweitert. Ist eine Handlungsanweisung Bestandteil der maskierten Mitteilung, so führt das empfangende Gerät diese aus. Mögliche Handlungen (und entsprechende auslösende Mitteilungen) sind in einer Datenbank im Speicher des Geräts abgelegt.

Alle Handlungsanweisungen können konditional mit Betriebsparametern der empfangenden Geräte und/oder zeitlich korreliert werden, d.h. eine Handlungsanweisung soll nach Ablauf einer bestimmten Zeit nach Empfang der Mitteilung ausgeführt werden und/oder wenn das empfangende Gerät sich in einem bestimmten Status befindet.

Das Senden und Empfangen der maskierten Töne in Verbindung mit den, dem Empfang nachgeschalteten, Handlungen ermöglicht eine mehrstufige Kommunikation zwischen mehreren Geräten ohne diese hardwareseitig ändern bzw. nachrüsten zu müssen. Die Kommunikation zwischen den Geräten soll bevorzugt parallel zu den bereits etablierten Standardtönen / Systemtönen ablaufen, kann aber auch bei Bedarf unmaskiert und / oder unabhängig von den Standardtönen / Systemtönen erfolgen.

Bei einem erfindungsgemäßen medizinischen Gerät ist die Ausgabeeinheit dazu ausgelegt, die Systemtöne und die Mitteilungstöne gleichzeitig auszusenden, sodass die Mitteilungstöne maskiert übermittelt werden.

Insbesondere kann seitens des medizinischen Geräts eine Steuerungseinheit vorgesehen sein, welche die Frequenz der von der Ausgabeeinheit auszusendenden Mitteilungstöne relativ zu der Frequenz der von der Ausgabeeinheit auszusendenden Systemtöne derart einstellt, dass eine Maskierung der Mitteilungstöne erfolgt.

Gemäß einem nicht beanspruchten Aspekt ist die Ausgabeeinheit dazu ausgelegt, die Mitteilungstöne unmittelbar vor oder nach den Systemtönen oder innerhalb von Systemtonpausen auszugeben.

Vorzugsweise ist die Steuerungseinheit dazu ausgelegt, die auszugebenden Mitteilungstöne vor der Ausgabe zu verschlüsseln bzw. zu maskieren.

Ein weitere Aspekt der Erfindung betrifft ein Verfahren zur Kommunikation eines medizinischen Geräts mit mindestens einer anderen Vorrichtung, mit den Schritten:
- Ausgeben eines vorzugsweise für das menschliche Gehör wahrnehmbaren Systemtones, vorzugsweise mittels einer Ausgabeeinheit des medizinischen Geräts und / oder der mindestens einen anderen Vorrichtung; und
- Ausgeben eines vorzugsweise für das menschliche Gehör nicht wahrnehmbaren Mitteilungstones, vorzugsweise mittels derselben Ausgabeeinheit des medizinischen Geräts und / oder der mindestens einen anderen Vorrichtung, wobei der Mitteilungston dazu ausgelegt ist, von dem medizinischen Gerät und / oder der mindestens einen anderen Vorrichtung empfangen zu werden.

Das Verfahren kann zur Kommunikation zwischen mehreren medizinischen Geräten oder zur Kommunikation eines medizinischen Gerät mit mindestens einer anderen, vorzugsweise nicht-medizinischen Vorrichtung, eingesetzt werden.

Zusätzlich zu Mitteilungen untereinander können die maskierten Töne der Geräte somit in anderen Worten auch von einem nicht-medizinischen Kommunikationsgerät bzw. einer Vorrichtung (z. B. einem Mobiltelefon) mit Mikrofon, Lautsprecher und gängiger Kommunikationsmöglichkeit (z. B. per WiFi) wahrgenommen werden.

Die nicht-medizinische Vorrichtung ist optimalerweise so in der Umgebung der medizinischen Geräte, z.B. im Behandlungsraum, positioniert, dass es die Töne aller im Raum befindlicher medizinischer Geräte störungsfrei wahrnehmen kann.

Vorzugsweise ist auch die nicht-medizinische Vorrichtung in der Lage, Töne, die von den medizinischen Geräten bzw. Behandlungsgeräten generiert werden, zu erzeugen. Durch die Netzwerkverbindung kann die nicht-medizinische Vorrichtung mit anderen nicht-medizinischen Geräten, z. B. mit einem Mobiltelefon des Pflegepersonals kommunizieren. Dadurch kann das Personal, auch wenn nicht in Hör- / Sichtweite zu den Behandlungsgeräten befindlich, über den Status von Geräteprozessen, etwaige Warnungen etc. informiert werden.

Umgekehrt kann auch das Personal per Eingabe an der nicht-medizinischen Vorrichtung / am Mobiltelefon Handlungsanweisungen an eine in der Umgebung der medizinischen Geräte befindliche nicht-medizinische Vorrichtung übermitteln, welche dann per Ton an einzelne medizinische Geräte übertragen werden. Auch eine direkte Kommunikation zwischen einem Kommunikationsgerät (z.B. ein Mobiltelefon) des Pflegepersonals und den medizinischen Geräten ist denkbar.

Das Verfahren umfasst weiterhin den Schritt:
- Maskieren des Mitteilungstones durch den Systemton, vorzugsweise mittels einer frequenzbasierten Maskierung oder, gemäß einem nicht beanspruchten Aspekt, einer zeitlichen Maskierung.

Die Maskierung bietet den Vorteil, dass menschliche Benutzer durch die Kommunikation der Geräte untereinander nicht abgelenkt oder gestört werden.

Gemäß einer vorteilhaften Ausführungsform umfasst das Verfahren weiterhin die Schritte:
- Auswerten des Mitteilungstones, vorzugsweise mittels einer Auswerteeinheit seitens des medizinischen Geräts und / oder der mindestens einen anderen Vorrichtung, und
- Ausführen eines in dem Mitteilungston codierten Steuerungsbefehls mittels einer Steuerungseinheit seitens des medizinischen Geräts und / oder der mindestens einen anderen Vorrichtung.

Bei dieser Ausführungsform wird das empfangende Gerät wie vorstehend beschrieben durch den Mitteilungston dazu veranlasst, eine vorbestimmte, in einer Datenbank abgelegte Handlung auszuführen.

Ein weiterer Aspekt der Erfindung betrifft ein System aus mindestens einem erfindungsgemäßen medizinischen Gerät sowie mindestens einer anderen Vorrichtung, insbesondere einer nicht-medizinischen Kommunikationsvorrichtung, wobei das System vorzugsweise dazu ausgelegt ist, eine Kommunikation gemäß eines erfindungsgemäßen Verfahrens auszuführen.

Vorzugsweise weist bei einem derartigen System die mindestens eine andere Vorrichtung eine Empfangseinheit, eine Ausgabeeinheit und vorzugsweise eine Einheit zur Netzwerkverbindung auf ist und vorzugsweise ein Mobiltelefon, ein Computer oder ein Tablet-Computer.

Ein anderer Aspekt der Erfindung betrifft ein Computerprogrammprodukt zur Nachrüstung eines herkömmlichen medizinischen Geräts, insbesondere einer extrakorporalen Blutbehandlungsvorrichtung, besonders eines Dialysegeräts, zur Ausführung eines erfindungsgemäßen Verfahrens, wobei das Computerprogrammprodukt Instruktionen enthält, welche, wenn sie von einem herkömmlichen medizinischen Gerät ausgelesen werden, dieses befähigen, ein erfindungsgemäßes Verfahren auszuführen.

In anderen Worten enthält ein erfindungsgemäßes Computerprogrammprodukt Instruktionen, mittels welcher ein herkömmliches medizinisches Gerät in ein erfindungsgemäßes medizinisches Gerät aufgerüstet bzw. nachgerüstet werden kann.

Das Computerprogrammprodukt kann beispielswiese auf einem Datenträger (USB-Stick, CD etc.) bereitgestellt werden. Alternativ kann das Computerprogrammprodukt auch in der Cloud bereitgestellt werden, sodass zur Nachrüstung eines bestimmten medizinischen Geräts zielgerichtet zur Aufrüstung dieses Geräts auf das in der Cloud abgelegte Programm zurückgegriffen werden kann.

Weitere Merkmale, Effekte und Vorteile der vorliegenden Erfindung ergeben sich aus der nachstehenden Beschreibung bevorzugter nicht- beschränkender Ausführungsbeispiele.

Dialysegeräte sollen in die Lage versetzt werden, miteinander oder mit weiteren externen Geräten zu kommunizieren, indem Informationen für das menschliche Ohr unhörbar in den Systemtönen untergebracht werden.

Da die Systemtöne eines Dialysegerätes aufgrund der Normenanforderungen sehr laut sein müssen, ergeben sich gute Maskierungseigenschaften auf den benachbarten "Frequenzbändern".

Hierbei sind mit Systemtönen alle herkömmlicherweise emittierten Töne eines medizinischen Geräts, z.B. Dialysegerätes gemeint, also z.B. Informations-, Meldungs- und Alarmtöne. Selbst das Testgeräusch beim T1-Test kann genutzt werden.

Eine Anwendungsmöglichkeit der vorliegenden Erfindung betrifft synchronisierte Abläufe mit mehreren Dialysegeräten.

Hierbei können Informationen in Meldungstönen z.B. benutzt werden, um eine Gruppe von Geräten, die sich z.B. im gleichen Raum befinden, synchronisiert, beginnend vom Einschalten aller Geräte, gemeinsam durch das Aufrüsten und Vorbereiten hindurch bis in die Behandlung zu steuern.

Dies ist besonders interessant, wenn sich die verschiedenen Geräte aufgrund unterschiedlicher Setupeinstellungen oder aufgrund unterschiedlicher Behandlungsverfahren unterschiedlich lange in den einzelnen Vorbereitungsschritten aufhalten.

Dadurch würde die Arbeit des Pflegepersonals deutlich erleichtert, wenn an allen Geräten immer zeitgleich bzw. nahezu gleichzeitig, die gleichen Handgriffe zu erledigen wären.

Die Synchronisation erfolgt hierbei derart, dass die Geräte dazu zu Beginn, d.h. mit dem Funktionstest, mit einem vorzugsweise maskierten Mitteilungston unter anderem ihre **ID** und ihren Standort an alle Geräte in der Umgebung versenden (Identifikationsmeldung). Wichtig ist nur, dass alle Geräte angeschaltet sind, wenn die Identifikationsmeldungen ertönen, sodass alle Geräte diese empfangen können

Alternativ findet zu Beginn eine Synchronisation statt. Dabei senden alle Geräte ein bestimmtes Signal aus und empfangen eintreffende Töne. Anschließend wird in den Geräten jeweils abgelegt, mit welchen und auch mit wie vielen anderen Geräten jedes Gerät verbunden ist und synchronisiert wird.

**In** anderen Worten wissen quasi alle Geräte, welche anderen Geräte mit ihnen in einem Raum stehen. Dazu wäre es notwendig, einem Gerät, z.B. beim Setup, seinen Standort mitzuteilen, damit es diesen aussenden kann.

Eine weitere Möglichkeit der Erkennung von Geräten in direkter Nähe wäre eine quantitative Analyse der Signalamplitude und -frequenz bei von den umliegenden Geräten empfangenen Mitteilungstönen. Eine Dämpfung durch Wände und andere Abtrennungen würden die Signalamplitude und -frequenz verändern.

Eine Information im Rahmen eines vorzugsweise maskierten Mitteilungstones übersandte Information könnte folgendermaßen aussehen: <Gerät xy, im Raum xyz, Behandlungsverfahren CVVxyz, Meldung #abcd (z.B. Beginn Füllen)>

Ein nächster Vorbereitungsschritt würde erst dann begonnen werden, wenn alle Geräte den dazu notwendigen Zustand erreicht hätten, z.B. alle Geräte den T1 Test bestanden hätten (und ein entsprechendes Signal aussenden) und mit dem Aufrüsten beginnen könnten, oder alle Geräte aufgerüstet sind und mit dem Füllen beginnen könnten.

Die Geräte müssen sich dafür zu Beginn gemerkt haben, welche Partnergeräte im Raum sind, damit sie wissen, ob alle Geräte den nächsten Schritt erreicht haben. Ein derartiges "Merken" erfolgt als Speichern einer entsprechenden Information in einer Datenbank. Die Geräte müssten dafür wissen bzw. gespeichert haben, welche Schritte im Rahmen der Vorbereitung bei den verschiedenen Behandlungsverfahren durchgeführt werden.

Sobald ein Gerät einen Zustand erreicht hat, sendet es einen einmaligen "Ich habe einen Zustand erreicht" -Mitteilungston aus und ist dann still. Wenn alle Geräte diesen Zustand erreicht haben, dann gibt das letzte Gerät den normalen Meldungston bzw. Systemton aus, und ein Anwender weiß Bescheid, dass alle Geräte den gleichen Zustand erreicht haben und der Anwender die nächste Aktion durchführen kann.

Grundsätzlich könnte auch eine zeitliche Beschränkung vorgesehen sein, damit, falls die Vorbereitungszeit an einem Gerät längerdauert, die restlichen Geräte nicht auch von der Verzögerung betroffen sind. Beispielsweise wird hierbei ein Zeitintervall festgelegt, in welchem sich ein Gerät mit der Beendigung eines Schrittes zu melden hat und ,wenn dies nicht mehr der Fall ist, wird noch einmal ein weiteres Intervall abgewartet und dann wird der nächste Schritt, eventuell ohne das verzögerte Gerät, gestartet.

Wenn ein Gerät in einen Fehlerzustand kommt, kann es dies einfach den anderen Geräten über einen Mitteilungston mitteilen. Dadurch könnten sich Geräte aus dem gemeinsamen Ablauf dynamisch an- und abmelden.

Das betroffene Gerät gibt darüber hinaus vorzugsweise einen hörbaren Signalton / Alarmton aus, um den Anwender zu rufen. Wenn die Verzögerungszeit zur Behebung des Alarms das festgelegte Zeitintervall nicht überschreitet, kann das Gerät auch wieder in die Synchronisation einsteigen.

Geräte könnten dabei einschätzen, ob ein anderes Gerät vor oder hinter ihnen im Ablauf steht.

Auf diese Weise könnten die Geräte aufeinander warten und könnten mit weniger Denkaufwand des Pflegepersonals in die Behandlung gebracht werden. Im Prinzip werden, im übertragenen Sinne (objektorientierte Programmierung), mehrere Instanzen von einen Gerät in die Behandlung gebracht.

Gemäß einer alternativen Lösung könnte es auch vorgesehen sein, dass mit einer Eingabe, z.B. der Betätigung eine Bedienfelds, an einem Gerät auch die anderen Geräte gestartet werden, sofern sie sich fehlerfrei an der gleichen Stelle im Ablauf befinden. Das hieße, ein Anwender könnte mit der Betätigung eine Bedienfelds an einem Gerät die gleiche Meldung gleichzeitig an allen Geräten bestätigen.

Dieses Kommando eines Gerätes an alle beteiligten Geräte kann auch in einem Mitteilungston übermittelt werden.

Eine weitere Anwendungsmöglichkeit betrifft die Information des Pflegepersonals via Mobiltelefon. Eine weitere Anwendungsmöglichkeit der Informationsübertragung via Mitteilungstöne besteht darin, dass das Pflegepersonal mittels ihrer Mobiltelefone diese Töne ebenfalls aufnehmen und auswerten könnte.

Dadurch könnte z.B. vermittelt werden, dass das Gerät xy im Raum xyz gerade die der Meldung bzw. den Status #abc ausgibt. Das Personal wüsste ohne die physische Anwesenheit im Raum, in welchem Zustand sich die Dialysegeräte gerade befinden.

Ein Pfleger würde also nicht nur von weitem sehen, dass das Dialysegerät xy rot blinkt, er könnte auch sehen, aufgrund welches Ereignisses dieses einen Alarm ausgibt.

Eine weitere Anwendungsmöglichkeit betrifft die Information des Pflegepersonals über ein Netzwerk. Hierbei könnten ein oder mehrere Zusatzgeräte (nicht-medizinische Vorrichtungen oder Kommunikationsgeräte), z.B. auch Mobiltelefone, ausgestattet mit einem Mikrofon, aufgestellt an zentralen Stellen auf der Station, die Systemtöne und die weiteren Informationen der Dialysegeräte, z.B. die Mitteilungstöne, aufnehmen und in ein Netzwerk einspeisen und damit die Informationen über alle Räume hinweg dem Pflegepersonal zugänglich machen.

Menschlichens Hörvermögen umfasst eine tiefste hörbare Frequenz von etwa 20 Hertz, die höchste hörbare Frequenz beträgt je nach Alter des Menschen maximal 20 kHz. Die Hörschwelle hängt von der Frequenz ab; die Wahrnehmungsempfindlichkeit ist bei etwa 4 kHz am höchsten. Die verwendeten Mitteilungstöne können für das menschliche Ohr unhörbar oder hörbar sein.

Grundsätzlich könnten Mitteilungstöne im unhörbaren Frequenzbereich ausgegeben werden. Bei manchen Hardware-Konfigurationen kommen nur hörbare Töne in Frage. Diese grundsätzlich hörbaren Töne können trotzdem für das menschliche Ohr unhörbar gemacht werden (Maskierung). Zwei Maskierungsarten sind bekannt:
Zum einen können mit Tönen im tiefen Frequenzbereich gleichzeitig Töne im mittleren Frequenzbereich verdeckt werden. Dieses Phänomen ist durch die menschliche Gehöranatomie bedingt.

Zum anderen gibt es noch die zeitliche Maskierung, bei der nach dem Ende eines relativ lauten Tones ein weiterer relativ leiserer Ton nicht wahrgenommen werden kann.

Ein Mikrofon hingegen kann diese Frequenzen dagegen wahrnehmen und durch entsprechende Bandpässe können somit verschiedene Frequenzbänder unterschiedlicher Signalleistung und somit SNR (Signal to Noise Ratio) festgelegt werden.

Mitteilungsinhalte können amplituden- oder frequenzmoduliert im Mitteilungston untergebracht werden.

Grundsätzlich wäre bei der Kommunikation ein Protokoll ähnlich dem CAN Bus vorstellbar. Es gibt eine Priorisierung der Nachrichten, d.h. die Nachrichten haben IDs, welche priorisiert sind. Dies ist notwendig, da alle Geräte auf demselben "Bus" d.h. derselben Frequenz / Leitung "funken"/senden. Dieses System hat den Vorteil, dass man Frequenzen identifizieren kann, welche zumindest teilweise nicht belegt sind, worüber die Geräte kommunizieren könnten.

Andersherum könnte man alternativ über einen Master jedem Gerät eine spezielle Frequenz erteilen.

Weiterhin sollte es auch eine Checksumme nach Empfang eines Mitteilungstons geben, womit die Nachricht auf Störungen untersucht werden kann. Die Checksumme ist ein Beispiel eines Verifizierungsschritts einer empfangenen Mitteilung.

Wird zudem mit dem Mitteilungston eine Identifikationskennung über eine Frequenz übertragen, wissen alle Geräte, welches Gerät senden darf und welches schweigen muss. Das heißt, danach kann man die Nutzinhalte / Nachrichten in weiteren Frequenzen übertragen, um schneller mehr Daten zu übertragen als rein über den Ton.

Unterschiedliche Übertragungsraten (BAUD Raten) auf unterschiedlichen Frequenzen sind hierbei möglich. Aus der Maximalstärke des übermittelten Signale ergibt sich ein Maximalabstand der Maschinen bzw. Geräte ergeben, in welchem Bereich die Geräte maximal entfernt zueinander kommunizieren können.

Um das Kommunikationsverfahren gegen Manipulationen abzusichern, sollten vorteilhafter Weise die Daten vor der Übermittlung verschlüsselt werden. Zusammen mit einem System zur Authentifizierung erhöht dies die Sicherheit der Kommunikation.

## Patentansprüche

1. Medizinisches Gerät, insbesondere Gerät zur extrakorporalen Blutbehandlung, mit einer Ausgabeeinheit zur Entsendung von Systemtönen zur Kommunikation mit einem Benutzer, wobei die Ausgabeeinheit zusätzlich dazu ausgelegt ist, Mitteilungstöne zur Kommunikation mit mindestens einer anderen Vorrichtung auszusenden und die Systemtöne und die Mitteilungstöne gleichzeitig auszusenden, sodass die Mitteilungstöne maskiert übermittelt werden.

2. Medizinisches Gerät nach Anspruch 1, **dadurch gekennzeichnet, dass** die Systemtöne für das menschliche Gehör wahrnehmbar sind und die Mitteilungstöne für das menschliche Gehör nicht wahrnehmbar sind.

3. Medizinisches Gerät nach Anspruch 1 oder 2, weiterhin mit einer Empfangseinheit, welche dazu ausgelegt ist, die Mitteilungstöne von mindestens einer anderen Vorrichtung zu empfangen und vorzugsweise zu entschlüsseln.

4. Medizinisches Gerät nach einem der vorhergehenden Ansprüche, weiterhin mit einer Steuerungseinheit, welche dazu ausgelegt ist, das medizinische Gerät in Übereinstimmung mit einem von der Empfangseinheit empfangenen Mitteilungston anzusteuern.

5. Medizinisches Gerät nach Anspruch 4, **dadurch gekennzeichnet, dass** die Steuerungseinheit, das medizinische Gerät dazu ansteuert, den empfangenen Mitteilungston zumindest teilweise auszugeben, einen vorbestimmten und vorzugsweise in einer Datenbank abgelegten Prozess zu initiieren oder zu beenden, Daten, insbesondere Daten bezüglich des Gerätezustands, als vorzugsweise maskierten Mitteilungston auszugeben, die Empfangseinheit auszuschalten oder Mitteilungstöne zu ignorieren oder einen für das menschliche Gehör wahrnehmbaren Ton zum Zwecke der Alarmierung auszugeben.

6. Medizinisches Gerät nach einem der vorherigen Ansprüche, weiterhin mit einer Steuerungseinheit, welche die Frequenz der von der Ausgabeeinheit auszugebenden Mitteilungstöne relativ zu der Frequenz der von der Ausgabeeinheit auszusendenden Systemtöne derart einstellt, dass eine Maskierung der Mitteilungstöne erfolgt.

7. Medizinisches Gerät nach Anspruch 4 oder 6, **dadurch gekennzeichnet, dass** die Steuerungseinheit dazu ausgelegt ist, die auszugebenden Mitteilungstöne vor der Ausgabe zu verschlüsseln.

8. Verfahren zur Kommunikation eines medizinischen Geräts gemäß einem der Ansprüche 1-7 mit mindestens einer anderen Vorrichtung, mit den Schritten:
- Ausgeben eines vorzugsweise für das menschliche Gehör wahrnehmbaren Systemtones, vorzugsweise mittels einer Ausgabeeinheit des medizinischen Geräts und / oder der mindestens einen anderen Vorrichtung;
- Ausgeben eines vorzugsweise für das menschliche Gehör nicht wahrnehmbaren Mitteilungstones, vorzugsweise mittels derselben Ausgabeeinheit des medizinischen Geräts und / oder der mindestens einen anderen Vorrichtung, wobei der Mitteilungston dazu ausgelegt ist, von dem medizinischen Gerät und / oder der mindestens einen anderen Vorrichtung empfangen zu werden; und Maskierung des Mitteilungstones durch den Systemton.

9. Verfahren nach Anspruch 8, wobei das -Maskieren des Mitteilungstones durch den Systemton mittels einer frequenzbasierten Maskierung erfolgt.

10. Verfahren nach Anspruch 8 oder 9, weiterhin mit den Schritten:
- Auswerten des Mitteilungstones, vorzugsweise mittels einer Auswerteeinheit seitens des medizinischen Geräts und / oder der mindestens einen anderen Vorrichtung, und
- Ausführen eines in dem Mitteilungston codierten Steuerungsbefehls mittels einer Steuerungseinheit seitens des medizinischen Geräts und / oder der mindestens einen anderen Vorrichtung.

11. System aus mindestens einem medizinischen Gerät gemäß einem der Ansprüche 1 bis 7 sowie mindestens einer anderen Vorrichtung, insbesondere einer nicht-medizinischen Kommunikationsvorrichtung, wobei das System vorzugsweise dazu ausgelegt ist, eine Kommunikation gemäß eines Verfahrens nach den Ansprüchen 8 bis 10 auszuführen.

12. System gemäß Anspruch 11, **dadurch gekennzeichnet, dass** die mindestens eine andere Vorrichtung eine Empfangseinheit, eine Ausgebeinheit und eine Einheit zur Netzwerkverbindung aufweist und vorzugsweise ein Mobiltelefon, ein Computer oder ein Tablet-Computer ist.

13. Computerprogrammprodukt zur Nachrüstung eines medizinischen Geräts, insbesondere einer extrakorporalen Blutbehandlungsvorrichtung, besonders eines Dialysegeräts, zur Ausführung eines Verfahrens gemäß einem der Ansprüche 8 bis 10, wobei das Computerprogrammprodukt Instruktionen enthält, welche, wenn sie von einem medizinischen Gerät ausgelesen werden, dieses befähigen, ein Verfahren gemäß einem der Ansprüche 8 bis 10 auszuführen.

14. Computerprogrammprodukt gemäß Anspruch 13, wobei das Computerprogrammprodukt Instruktionen enthält, welche, wenn sie von einem herkömmlichen medizinischen Gerät ausgelesen werden, diesem die Merkmale eines medizinischen Geräts gemäß einem der Ansprüche 1 bis 7 verleihen.

## Claims

1. Medical device, in particular a device for extracorporeal blood treatment, having an output unit for outputting system sounds for communication with a user, wherein the output unit is additionally adapted to transmit message sounds for communication with at least one other apparatus and to simultaneously emit the system sounds and the message sounds so that the message sounds are transmitted in a masked manner.

2. Medical device in accordance with claim 1, **characterized in that** the system sounds are perceptible to human hearing and the message sounds are not perceptible to human hearing.

3. Medical device in accordance with claim 1 or claim 2, further comprising a reception unit that is configured to receive and preferably to decrypt the message sounds from at least one other apparatus.

4. Medical device in accordance with one of the preceding claims, further comprising a control unit that is adapted to control the medical device in agreement with a message sound received by the reception unit.

5. Medical device in accordance with claim 4, **characterized in that** the control unit controls the medical device to at least partially output the received message sound, to initiate or end a predetermined process preferably stored in a database, to output data, in particular data with respect to the device status, as a preferably masked message sound, to switch off the reception unit or to ignore message sounds or to output a sound perceptible to human hearing for the purpose of emitting an alarm.

6. Medical device in accordance with one of the preceding claims, further comprising a control unit that sets the frequency of the message sounds to be outputted by the output unit relative to the frequency of the system sounds to be transmitted by the output unit such that a masking of the message sounds takes place.

7. Medical device in accordance with claim 4 or 6, **characterized in that** the control unit is adapted to encrypt the message sounds to be output prior to the output.

8. Method of communication of a medical device in accordance with one of the claims 1-7, with at least one other apparatus, comprising the steps:
- outputting a system sound preferably perceptible to human hearing, preferably by means of an output unit of the medical device and/or of the at least one other apparatus;
- outputting a message sound preferably not perceptible to human hearing, preferably by means of the same output unit of the medical device and/or of the at least one other apparatus, with the message sound being adapted to be received by the medical device and/or the at least one other apparatus; and masking of the message sound with the system sound.

9. Method in accordance with claim 8, wherein masking the message sound with the system sound takes place by means of a frequency-based masking.

10. Method in accordance with claim 8 or claim 9, further comprising the steps:
- evaluating the message sound, preferably by means of an evaluation unit at the side of the medical device and/or of the at least one other apparatus; and
- performing a control command encoded in the message sound by means of a control unit at the side of the medical device and/or of the at least one other apparatus.

11. System of at least one medical device in accordance with one of the claims 1 to 7, and at least one other apparatus, in particular a non-medical communication apparatus, wherein the system is preferably adapted to perform a communication in accordance with a method in accordance with claims 8 to 10.

12. System in accordance with claim 11, **characterized in that** the at least one other apparatus has a reception unit, an output unit, and a unit for a network connection and is preferably a cellular phone, a computer, or a tablet computer.

13. Computer program product for upgrading a medical device, in particular an extracorporeal blood treatment apparatus, particularly a dialysis machine, for carrying out a method in accordance with one of the claims 8 to 10, wherein the computer program product contains instructions that, when they are read out by a medical device, enable it to carry out a method in accordance with one of the claims 8 to 10.

14. Computer program product in accordance with claim 13, wherein the computer program product contains instructions that, when they are read out by a conventional medical device, provide it with the features of a medical device in accordance with one of the claims 1 to 7.

## Revendications

1. Appareil médical, notamment appareil de traitement extracorporel du sang, avec une unité d'émission pour émettre des tonalités de système pour communiquer avec un utilisateur, l'unité d'émission étant en outre conçue pour émettre des tonalités de message pour communiquer avec au moins un autre dispositif et pour émettre les tonalités de système et les tonalités de message simultanément, de telle sorte que les tonalités de message sont transmises de manière masquée.

2. Appareil médical selon la revendication 1, **caractérisé en ce que** les tonalités de système sont perceptibles pour l'oreille humaine et les tonalités de message ne sont pas perceptibles pour l'oreille humaine.

3. Appareil médical selon la revendication 1 ou 2, avec en outre une unité de réception conçue pour recevoir et, de préférence, décoder les tonalités de message d'au moins un autre dispositif.

4. Appareil médical selon l'une quelconque des revendications précédentes, avec en outre une unité de commande conçue pour commander l'appareil médical en fonction d'une tonalité de message reçue par l'unité de réception.

5. Appareil médical selon la revendication 4, **caractérisé en ce que** l'unité de commande commande l'appareil médical pour émettre au moins partiellement la tonalité de message reçue, pour lancer ou arrêter un processus prédéterminé et de préférence stocké dans une base de données, pour émettre des données, notamment des données relatives à l'état de l'appareil, sous forme de tonalité de message de préférence masquée, pour désactiver l'unité de réception ou pour ignorer des tonalités de message ou pour émettre une tonalité perceptible par l'oreille humaine à des fins d'alarme.

6. Appareil médical selon l'une quelconque des revendications précédentes, avec en outre une unité de commande qui règle la fréquence des tonalités de message à émettre par l'unité d'émission par rapport à la fréquence des tonalités de système à émettre par l'unité d'émission de telle sorte qu'un masquage les tonalités de notification soit effectué.

7. Appareil médical selon la revendication 4 ou 6, **caractérisé en ce que** l'unité de commande est conçue pour coder les tonalités de message à émettre avant l'émission.

8. Procédé de communication d'un appareil médical selon l'une quelconque des revendications 1 à 7 avec au moins un autre dispositif, avec les étapes suivantes :
- l'émission d'une tonalité de système, de préférence perceptible par l'oreille humaine, de préférence au moyen d'une unité d'émission de l'appareil médical et/ou de l'au moins un autre dispositif ;
- l'émission d'une tonalité de message, de préférence non perceptible par l'oreille humaine, de préférence au moyen de la même unité d'émission de l'appareil médical et/ou de l'au moins un autre dispositif, la tonalité de message étant conçue pour être reçue par l'appareil médical et/ou l'au moins un autre dispositif ; et
le masquage de la tonalité de message par la tonalité de système.

9. Procédé selon la revendication 8, dans lequel le masquage de la tonalité de message par la tonalité de système est effectué au moyen d'un masquage basé sur la fréquence.

10. Procédé selon la revendication 8 ou 9, avec en outre les étapes suivantes :
- l'évaluation de la tonalité de message, de préférence au moyen d'une unité d'évaluation au niveau de l'appareil médical et/ou de l'au moins un autre dispositif, et
- la mise en œuvre d'une instruction de commande codée dans la tonalité de message au moyen d'une unité de commande au niveau de l'appareil médical et/ou de l'au moins un autre dispositif.

11. Système composé d'au moins un appareil médical selon l'une quelconque des revendications 1 à 7, ainsi que d'au moins un autre dispositif, notamment un dispositif de communication non médical, le système étant de préférence conçu pour mettre en œuvre une communication par un procédé selon les revendications 8 à 10.

12. Système selon la revendication 11, **caractérisé en ce que** l'au moins un autre dispositif présente une unité de réception, une unité d'émission et une unité de connexion au réseau et est de préférence un téléphone mobile, un ordinateur ou une tablette.

13. Produit de programme d'ordinateur pour la mise à niveau d'un appareil médical, notamment d'un dispositif de traitement extracorporel du sang, notamment d'un appareil de dialyse, pour la mise en œuvre d'un procédé selon l'une quelconque des revendications 8 à 10, le produit de programme d'ordinateur contenant des instructions qui, lorsqu'elles sont lues par un appareil médical, permettent à celui-ci de mettre en œuvre un procédé selon l'une quelconque des revendications 8 à 10.

14. Produit de programme d'ordinateur selon la revendication 13, le produit de programme d'ordinateur contenant des instructions qui, lorsqu'elles sont lues par un appareil médical conventionnel, lui confèrent les caractéristiques d'un appareil médical selon l'une quelconque des revendications 1 à 7.
